## Europäisches Patentamt

## European Patent Office

## Office européen des brevets

(11) Veröffentlichungsnummer: **0 189 378**
**B1**

(12) **EUROPÄISCHE PATENTSCHRIFT**

(45) Veröffentlichungstag der Patentschrift:
31.05.89

(51) Int. Cl.⁴: **C 12 J 1/10, C 12 M 1/12**

(21) Anmeldenummer: **86810040.5**

(22) Anmeldetag: **24.01.86**

(54) **Verfahren und Anlage zur submersen, kontinuierlichen Herstellung von Gärungsessig aus Aethanol.**

(30) Priorität: **25.01.85 CH 335/85**

(43) Veröffentlichungstag der Anmeldung:
**30.07.86 Patentblatt 86/31**

(45) Bekanntmachung des Hinweises auf die Patenterteilung:
**31.05.89 Patentblatt 89/22**

(84) Benannte Vertragsstaaten:
**AT BE CH DE FR GB IT LI LU NL SE**

(56) Entgegenhaltungen:
**FR-A-2 160 853**
**US-A-4 456 622**

**CHEMICAL ABSTRACTS, Band 93, Nr. 7, August 1980, Seite 764, Nr. 69069s, Columbus, Ohio, US; & JP - A - 80 08 150**
**CHEMICAL ABSTRACTS, Band 93, Nr. 9, September 1980, Seite 518, Nr. 93827h, Columbus, Ohio, US; & JP - A - 80 54 890**
**PATENTS ABSTRACTS OF JAPAN, Band 4, Nr. 93 (C-17) 575 , 5. Juli 1980; & JP - A - 55 54 890**

(73) Patentinhaber: **Heinrich Frings GmbH & Co. KG, Jonas- Cahn- Strasse 9, D-5300 Bonn 1 (DE)**

(72) Erfinder: **Aeschbach, Hermann, Dr.sc.techn., 8, chemin Vers- chez- Cottier, CH- 1807 Blonay (CH)**

(74) Vertreter: **Frauenknecht, Alois J., c/o PPS Polyvalent Patent Service AG Mellingerstrasse 1, CH- 5400 Baden (CH)**

**Beschreibung**

Die Erfindung betrifft ein Verfahren zur submersen, kontinuierlichen Herstellung von Gärungsessig aus Äthanol in wenigstens zwei Stufen, wobei die den Essig bildenden Mikroorganismen ausserhalb eines Bioreaktors von der vergorenen Maische mittels Membranfilter abgetrennt werden sowie eine Vorrichtung zur Durchführung des Verfahrens.

Es ist ein Verfahren zur Abtrennung von Mikroorganismen aus einer vergorenen Maische mittels Membranfiltration aus der US-PS-3 974 068 bekannt. Zur Unterdrückung der Konzentrationspolarisation, verursacht durch die im Laufe der Filtration ständig ansteigenden Menge an Essigbakterien, wird sowohl bei hohen Umpumpgeschwindigkeiten als auch unter Zusatz von Filterhilfsmitteln gearbeitet.

Nach den bekannten Trennverfahren, wobei der Einsatz eines Membranfilters getrennt vom mikrobiologischen Prozess erfolgt, ist es nicht möglich, Essigbakterien am Leben zu erhalten, da diese ohne Unterbruch ausreichend mit Sauerstoff versorgt werden müssen. Auch durch die hohen Umpumpgeschwindigkeiten und die Dauer der Umwälzung während der Ultrafiltration werden die empfindlichen Bakterien durch auftretende Scherkräfte geschädigt. Der Zusatz von Filterhilfsmitteln ist unerwünscht bei einer Rückführung in den Fermenter, da diese einen störenden Fremdkörper darstellen. Filterhilfsmittel stellen eine zusätzliche Aufwendung an Material dar, welche das Verfahren in seiner Wirtschaftlichkeit beeinträchtigt und welche schliesslich wieder beseitigt werden müssen.

Es ist ferner bekannt (US-PS-4 456 622), hochprozentigen Essig in einer Konzentration von 18 - 20 Vol.-% in zwei Fermentationsstufen herzustellen. In der ersten Stufe erfolgt eine semikontinuierliche Versäuerung des Substrat-Äthanols auf 10 - 12 Vol.-% und in der zweiten Stufe eine Versäuerung auf 18 - 20 Vol.-% bei Chargenbetrieb. Es resultieren dabei Produktionsraten in der Grössenordnung von max. 1,5 g/l h⁻¹. Die geringe Produktivität resultiert u. a. durch eine in der zweiten Verfahrensstufe notwendige energieaufwendige Kühlung des Fermenterinhaltes auf eine um 8 - 12°C tiefere Temperatur. Dieses Verfahren ist deshalb nicht wirtschaftlich und zudem durch den notwendigen Chargenbetrieb in seiner Leistung sehr begrenzt.

Aufgabe der Erfindung ist es, ein Verfahren zu schaffen, welches unter mikrobiologisch optimalen Bedingungen eine industriegerechte und wirtschaftliche kontinuierliche Herstellung von Gärungsessig mit wenigstens 120 g/l Essigsäure ermöglicht, welche eine mindestens dreifach höhere Produktivität als beim anfangs beschriebenen Verfahren und der Anlage erzielt und das Substrat bis zur maximal möglichen durch die Acetobacter erreichbaren Ausbeute umsetzt.

Diese Aufgabe wird erfindungsgemäss dadurch gelöst, dass die aktiven Mikroorganismen im Retentat unter Begasung unmittelbar nach dem Mikro/Ultrafilter in den Bioreaktor zurückgeführt und in hoher Konzentration von bis zu 0,3 g/l angereichert werden und mittels eines zugeschalteten Hyperfilters der erzeugte Gärungsessig wenigstens partiell als Endprodukt abgetrennt und durch Hyperfiltration die Konzentration an Essigsäure im Bioreaktor gesteuert wird und das Verfahren so durchgeführt wird, dass der aus dem Mikro/Ultrafilter austretende Gärungsessig mittels eines Hyperfilters bei 40 - 80 bar und 28 - 32°C auf etwa 120 g/l Essigsäure konzentriert wird und dass das Medium dem Bioreaktor im Bereich der Begasungszone eines Rührers entnommen wird.

Bioreaktor und Mikro/Ultrafilter sind so miteinander verknüpft, dass zwischen ersterem und dem Konzentratraum des Mikro/Ultrafilters nur ein kinetisch definierter Mikroorganismenaustausch nach aussen stattfinden kann. Als Mikrofilter werden handelsübliche Mikroporenfilter verstanden, welche eine Porengröse von 10 - 0,1 µm aufweisen; als Ultrafilter solche, deren Membranen eine Porengrösse von 0,1 µm bis 0,001 µm aufweisen. Besonders vorteilhaft sind sogenannte Mikrotangentialfilter. Durch die kontinuierliche Abführung mindestens eines Teils des produzierten Gärungsessigs aus dem Bioreaktor, mittels nachgeschalteter Hyperfiltration, kann sowohl die Mikroorganismenkonzentration als auch die Essigkonzentration im Bioreaktor auf einem konstanten Wert gehalten, gesteuert und damit die Produktinhibition durch ansteigende Essigsäurekonzentration herabgesetzt werden. Die Rückführung erfolgt durch ununterbrochene Begasung der Essigbakterien auf ihrem Weg vom Bioreaktor über den Membranfilter und hält diese vital, d. h. ihre Vermehrung wird nicht gehemmt.

Das Verfahren wird vorzugsweise zweistufig ausgeführt, wobei in einer ersten Gärungsstufe der Bioreaktor unter kinetisch optimalen Verhältnisses kontinuierlich betrieben wird und in einer zweiten Stufe das Substrat bis zur vollständigen Umsetzung des Äthanols zu Essigsäure versäuert wird.

Die Zellanreicherung im Bioreaktor erreicht eine 4 bis 5-fache Konzentration gegenüber bekannten Verfahren, wodurch mindestens dreifach höhere Produktionsraten als bei dem anfangs beschriebenen bekannten Prozess bei ausreichender Sauerstoffversorgung resultieren.

Es hat sich als besonders vorteilhaft erwiesen, das Medium dem Bioreaktor auf der Höhe der Begasungszone des Rührers, d. h. im radialen Begasungsbereich der Turbine, zu entnehmen, da dort die Sauerstoffkonzentration der Flüssigkeit am höchsten ist. In diesem Zustand reicht bei Industrieanlagen die Konzentration an Sauerstoff aus, um die Bakterien auf dem Weg durch den Mikro/Ultrafilter mit genügend Sauerstoff zu versorgen.

Darüber hinaus ist es, gemäss Anspruch 3, zweckmässig, sogleich nach dem Austritt aus dem Mikro/Ultrafilter falls erforderlich erneut zu begasen, was unmittelbar vor und nach oder in der Pumpe, beispielsweise mit an Sauerstoff angereicherter Luft, geschieht. Durch die Verwendung von sauerstoffreicher Luft kann die total erforderliche Luftmenge gering gehalten werden.

Es kann sogar zweckmässig sein, gemäss Anspruch 4, eine weitere Begasung vor dem Eintritt in den Bioreaktor vorzunehmen, vor allem dann, wenn der Weg zwischen der Pumpe und dem Bioreaktor aus Anordnungsgrönden länger gewählt werden muss. Die gesamte Rezirkulationsdauer ist sehr kurz und beträgt, gemäss Anspruch 5, weniger als 15 Sekunden.

Gemäss Anspruch 6 ist es zweckmässig, den Abfluss des Permeats intermittierend mit Pressluft für wenige Sekunden zu unterbrechen und in entgegengesetzter Richtung durch die Membranen zu drücken, um rückzuspülen und so die Standzeit der Membranen durch deren Abreinigung weiter zu erhöhen.

Es ist vorteilhaft, gemäss Anspruch 7, das Permeat der Mikro- bzw. Ultrafiltration einer nachgeschalteten Hyperfiltration zu unterziehen. Es ist zweckmässig diese Behandlung bei einem Betriebsdruck von 40 bis 80 bar, insbesondere bei 57 bis 60 bar, und einer Temperatur von 28 bis 32°C durchzuführen. Unter diesen Bedingungen wird das Permeat aus der Ultrafiltration auf wenigstens 120 g/l Essigsäure aufkonzentriert.

Gemäss Anspruch 8 ist es zweckmässig, das Permeat aus der Hyperfiltration, welches etwa 25 bis 35 g/l Restsäure enthält, in den Bioreaktor über die Substratzufuhr zurückzuführen und diese Säuremenge bei der Substratzufuhr entsprechend zu berücksichtigen.

Eine Anlage zur Durchführung des Verfahrens besteht in ihren wesentlichen Teilen aus wenigstens zwei Bioreaktoren, die die Hauptstufe und die Erntestufe bilden, niveaugesteuerten Dreiweg-Magnetventilen, einem Mikro- oder Ultrafilter und einem Hyperfilter.

Es ist, gemäss Anspruch 9, zweckmässig, den Bioreaktor in einer ersten Stufe als Chemostat unter kinetisch optimalen Verhältnissen zu betreiben und in einer zweiten Stufe, in einem Erntefermenter, bis zur vollständigen Umsetzung des Äthanols zu Essigsäure, zu verarbeiten.

Es ist, gemäss Anspruch 8, wesentlich, dass ein Bioreaktor lediglich unter Zwischenschaltung eines Dreiwegventils unmittelbar mit einem begasbaren Membranfilter verbunden ist. Im Membranfilter ist eine Pumpe in bekannter Weise integriert. Die Membranen des Filters weisen bevorzugt einen Porendurchmesser von max. 0,65 µm auf, wobei bei einem Betriebsdruck von etwa 0,5 bis 4 bar vorzügsweise 0,5 bar filtriert wird. Das Medium im Membranfilter wird auf der Konzentratseite ständig mit Sauerstoff angereicherter Luft begast.

Als Begasungseinrichtungen haben sich, gemäss Anspruch 9, Diffusoren bewährt, wobei ein Begasungsdiffusor vor dem Mikro/Ultrafilter und ein Begasungsdiffusor bzw. Injektor vor einer Pumpe und ein weiterer Begasungsdiffusor nach einer Pumpe vorgesehen ist. Das hat den Vorteil, dass für den einzelnen Diffusor nur geringe Luftmengen erforderlich sind und dennoch kein Unterbruch der Sauerstoffzufuhr eintreten kann, auch dann nicht, wenn ein Diffusor ausfallen sollte.

Das Verfahren soll beispielhaft anhand von Zeichnungen näher erläutert werden.

Es zeigen:

Fig. 1    ein Schema einer Laborapparatur zur Herstellung von Gärungsessig nach dem erfindungsgemässen Verfahren,

Fig. 2    ein Schema für eine Zellrückführung und eine Produktabtrennung,

Fig. 3    ein Schema einer Industrieanlage und

Fig. 4    ein Schema eines Diffusors.

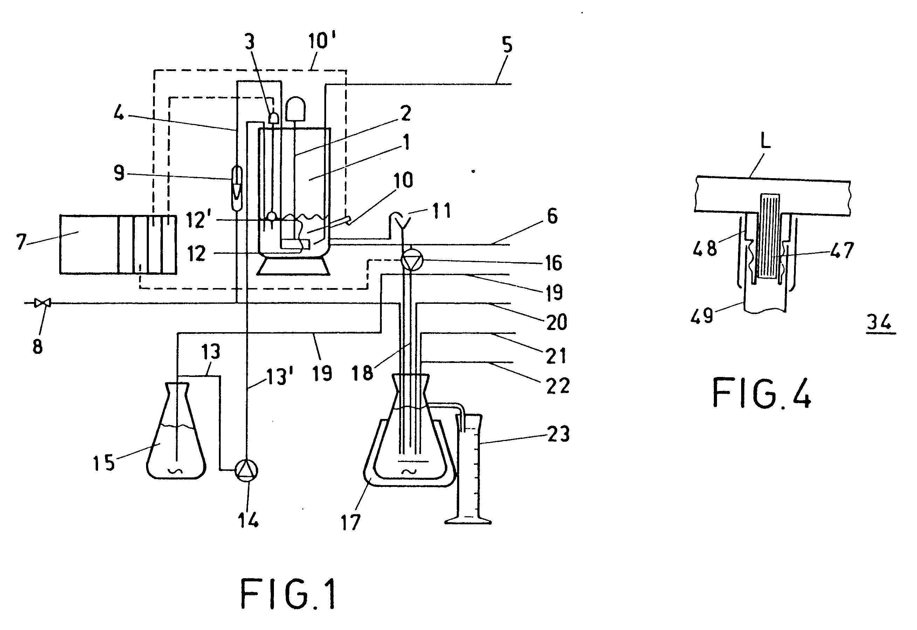

Fig. 1 zeigt eine Versuchsanlage für eine kontinuierliche Kultur sowie für eine Satzkultur mit Teilerneuerung, die im einfachsten Fall aus einem bekannten Bioreaktor 1 besteht. Der Bioreaktor 1 stellt einen Behälter mit den erforderlichen Anschlüssen dar, in welchem die für die Erfindung wesentlichen Teile in der Figur eingezeichnet sind. Ein Rührer 2 ist axial-zentral, und eine Einrichtung zur Steuerung des Niveaus 3 ist gleichfalls axial installiert. Eine Leitung 4 dient der Zufuhr von Druckluft zur Begasung des Mediums und mündet im Bereich der Rührflügel des Rührers 2. In der Begasungszone des Rührers 2 ist eine Leitung 5 installiert und im Boden des Behälters eine weitere Leitung 6. Der Bioreaktor 1 ist mit einem Steuerschrank 7 über diverse Steuerleitungen verbunden. Mit 8 ist eine Verteilerstation für Druckluft bezeichnet, welche einen Druckregler und eine Filtereinheit für Druckluft enthält. In der Leitung 4 ist ein Rotameter 9 zur Gasdurchflussmessung vorgesehen. Eine Sauerstoffsonde 10 ist über ein Sondenkabel 10' mit dem Steuerschrank 7 verbunden. Für einfachen kontinuierlichen Betrieb ist ein Niveauüberlauf 11 vorgesehen. Der Bioreaktor 1 ist zur Kühlung mit Anschlussleitungen 12 und 12' für den Eintritt und Austritt eines Wärmeübertragungsmediums versehen. Eine Leitung 13 für die Substratzufuhr wird über eine Pumpe 14 mit Medium aus einem Substrataufbereitungsgefäss 15 beschickt. In einem Abzweig der Leitung 6 ist eine Erntepumpe 16 vorgesehen, welche über eine Leitung 18 mit einem luftbegasten thermostatisierten Erntefermenter 17 verbunden ist. Der Erntefermenter 17 ist mit dem Überlaufgefäss 23 verbunden.

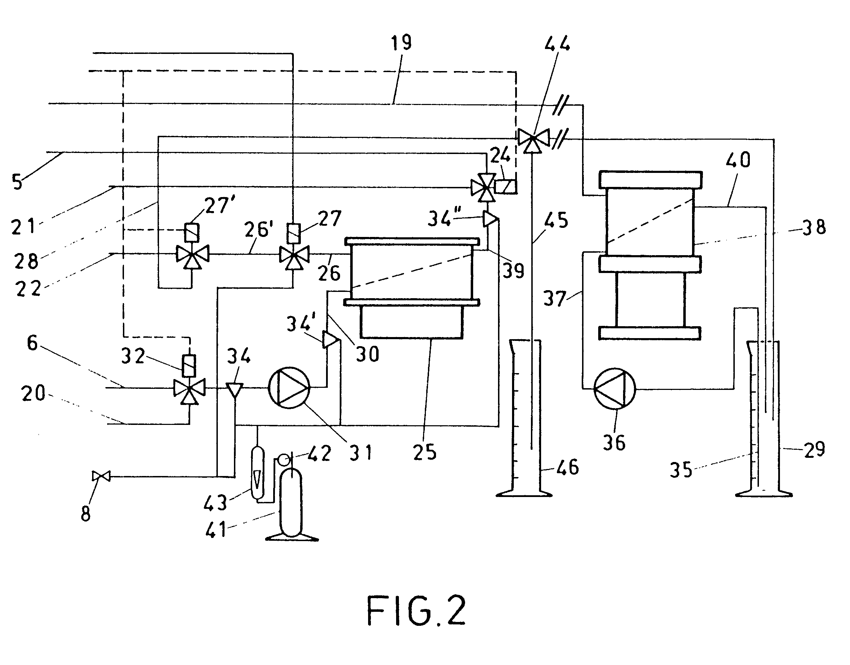

In Fig. 2 wird der Teil der Laborapparatur gezeigt, welcher über die Leitungen 5, 6, 19, 20, 21 und 22 mit der Apparatur gemäss Fig. 1 verbunden ist. Die Leitung 6 führt über ein Dreiweg-Magnetventil 32 auf ein Mikro/Ultrafilter 25, welcher einen Mikrotangentialfilter mit Membranpumpe darstellen kann. Die Membranen des Mikro/Ultrafilters 25 sind durch eine gestrichelt gezeichnete Diagonale symbolisiert. Im oberen Teil des Mikro/Ultrafilters 25 führt eine Leitung 26 über ein Dreiweg-Magnetventil 27 für die automatische Rückspülung mit Druckluft und ein weiteres Dreiweg-Magnetventil 27' zur niveaugesteuerten Umschaltung des Permeatflusses, entweder über die Leitong 22 oder über eine Leitung 28 in ein Produktgefäss 29. Von der Leitung 28 führt über ein Dreiwegventil 44 eine Leitung 45 in einen Behälter 46 zur Aufnahme des unkonzentrierten Permeats aus dem Mikro/Ultrafilter 25.

Im oberen Teil des Mikro/Ultrafilters 25 führt eine Leitung 39 über ein weiteres Dreiweg-Magnetventil 24 und die Leitung 21 in den gerührten, luftbegasten und thermostatisierten Erntefermenter 17 (Fig. 1).

Mit den Bezugszeichen 34, 34', 34" werden Begasungsdiffusoren für $O_2$- angereicherte Luft beschrieben. Aus dem Produktgefäss 29 führt eine Leitung 35 auf eine Hochdruckpumpe 36, eine Druckleitung 37 und auf das Hyperfilter 38. Vom Hyperfilter 38 führt eine Permeatleitung 19 über das Substrataufbereitungsgefäss 15 zurück zum Bioreaktor 1. Eine Konzentratleitung 40 führt zum Produktgefäss 29.

Eine Sauerstoffdosiereinheit zur $O_2$-Anreicherung der Druckluft für die Begasung während der Filtration besteht aus einer Sauerstofffasche 41 mit einem Manometer und einem Druckreduzierventil 42 sowie einem Rotameter 43.

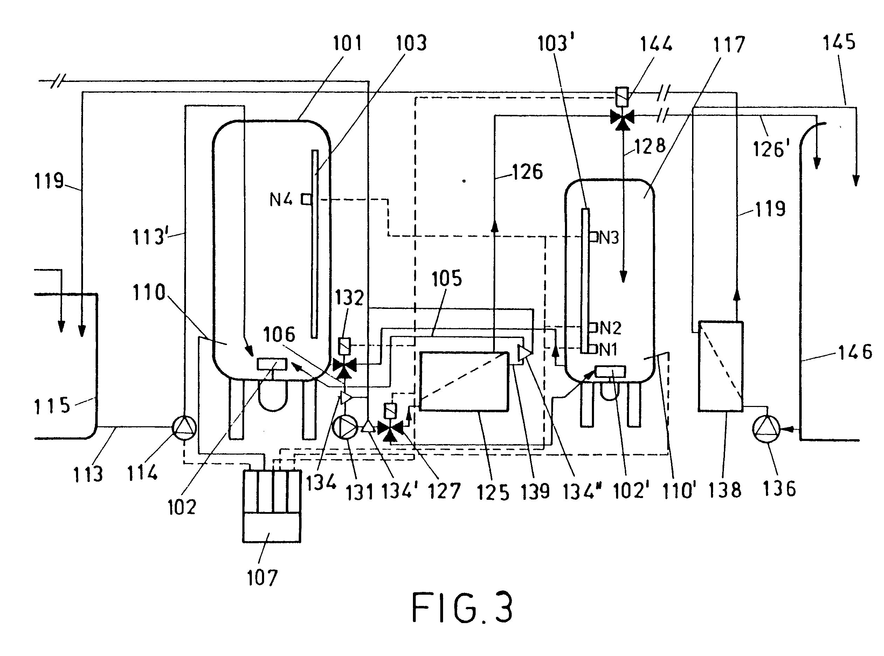

Fig. 3 zeigt ein Schema für ein industrielles Zweistufenverfahren. Die Bezugsziffern entsprechen denjenigen der Fig. 1 und 2 und unterscheiden sich lediglich durch eine vorgesetzte 1. Die Verbindungsleitungen wurden der Übersicht wegen nur teilweise bezeichnet. Mit 101 ist ein Bioreaktor bezeichnet. Der Bioreaktor 101 dient für den Chemostatbetrieb und ist mit seinen für die Erfindung wesentlichen Teilen versehen, einem Rührer 102, einer Niveausteuerung 103 mit einem Kontakt N4 und einer Sauerstoffsonde 110. Die Steuerung erfolgt über einen Steuerschrank 107. Ein Substrataufbereitungsbehälter 115 ist über eine Leitung 113, eine Pumpe 114 und einen Leitungsteil 113' mit dem Bioreaktor 101 verbunden. Ein Erntefermenter 117 ist mit einem Rührer 102', einer Sauerstoffsonde 110' und einer Niveausteuerung 103' versehen, welche die Niveauanzeigen N1, N2 und N3 aufweist. In einer Ernteleitung 106 ist ein Dreiwegventil 132, ein Diffusor 134, eine Pumpe 131, ein weiterer Diffusor 134', ein Dreiwegventil 127 eingebaut und mit einem Mikro/Ultrafilter 125 verbunden. Ein weiterer Diffusor 134" ist in der Rückleitung 105 des Retentats zum Bioreaktor 101 vorgesehen. Die Gasleitungen sind auch hier der Übersichtlichkeit wegen nicht mit Bezugsziffern bezeichnet. Der Mikro/Ultrafilter 125 ist über eine Leitung 126 und ein Dreiwegventil 144 mit dem Produktbehälter 146 verbunden. Vom Dreiwegventil 144 führt eine Leitung 128 in den Erntefermenter 117 und eine Leitung 126' in den Produktbehälter 146. Der Produktbehälter 146 ist über eine Hochdruckpumpe 136 mit dem Hyperfilter 138 verbunden. Die Leitung 119 des Hyperfilters 138 führt in den Substrataufbereitungsbehälter 115, die Konzentratleitung 145 in den Produktbehälter 146.

In Fig. 4 ist der erfindungsgemäss verwendete Diffusor 34 schematisch dargestellt. Er besteht in seiner einfachsten Ausführung aus einem porösen Körper 47, der mittels einer Klemmeinrichtung 48 innerhalb eines Gaszufuhrrohrs 49 befestigt ist. Der poröse Körper 47 besteht aus Holz, vorzugsweise aus Lindenholz oder Porzellan, und ist in den Leitungsquerschnitt einer Flüssigkeit führenden Leitung L in vorgeschobener Lage fixiert.

Während des Betriebs des Begasungsdiffusors strömt in der Leitung L zu begasendes Medium, sowie Luft oder mit Sauerstoff angereicherte Luft, von einer nicht gezeigten Druckgasquelle über das Rohr 49 in den porösen Körper 47 und gibt das Gas in feinen Blasen an die strömende Flüssigkeit ab.

Im Betrieb der Laboranlage wird ein Medium mit Bakterien einer Acetobacter-Art im Bioreaktor 1 unter Umwälzung mittels des selbstansaugenden Rührers 2 mit 0,1 bis 1 vvm über die Leitung 4 begast. Gleichzeitig wird eine stetige Menge an Medium im Bereich der Begasung am Anschluss der Leitung 6 entnommen, über das in Richtung des Mikro/Ultrafilters 25 offene Dreiwegventil 32 geführt, wobei die Pumpe 31 für das nötige Druckgefälle von ca. 0,5 bar sorgt. Gleichzeitig wird wenn erforderlich über den Begasungsdiffusor 34 mit Sauerstoff angereicherte Luft unter einem Druck von mit 0,6 bar in die Zufuhrleitung zum Mikro- oder Ultrafilter 25 dosiert. Das Permeat mit von Bakterien freier Essigsäure fliesst über die Leitung 26, des Dreiwegventils 27, den Leitungsteil 26', das Dreiwegventil 27' über die Leitung 22 in das Erntegefäss 17 oder in das Produktgefäss 29, in welchem der Essig mit einer Konzentration von ca. 60 g/l Essigsäure aufgefangen wird. Das Konzentrat, welches die vitalen Essigbakterien enthält, wird unmittelbar vor dem Eintritt in die Pumpe 31 mittels des Diffusors 34 begast, nochmals nach der Pumpe 30 durch den Diffusor 34' und über das Dreiwegventil 24 und die Leitung 5 unmittelbar in den Bioreaktor 1 zurückgeführt. Eine weitere Begasung kann unmittelbar nach dem Membranfilter 25 mittels des Diffusors 34" erfolgen. Es ist aber auch möglich, die Begasung in der Pumpe 31 auszuführen. Die Durchlaufzeit des Mediums beträgt weniger als 15 Sekunden. Durch die fast ununterbrochene Begasung werden die rückgeführten Bakterien in ihrer Produktivität nicht nachteilig beeinflusst.

Zur Aufkonzentrierung der Essigsäure wird dem Produktgefäss 29 über die Leitung 35 mittels der Hochdruckpumpe 36 Essiglösung (60 g/l Essigsäure) entnommen und über die Druckleitung 37 durch die Membranen des Hyperfilters 38 und die Leitung 40 so lange umgepumpt, bis das Volumen auf etwa 1/3 verringert ist und so die gewünschte Konzentration an Essigsäure von wenigstens 120 g/l erreicht ist. Das Permeat des Hyperfilters 38 wird über die Leitung 19 dem Substrataufbereitungsgefäss 15 zugeführt. Die Abstimmung der Hyperfiltration mit der Ultrafiltration und der Fermentation kann so erfolgen, dass in Funktion der vom Bioreaktor 1 gegebenen Flussrate die Membranfilterleistungen ausgelegt werden und dem Produktgefäss 29 bei entsprechend maximaler Grenzkonzentration Fertigessig entnommen wird.

**Beispiel**

Als Essigbildner wurde ein bekannter an Betriebsbedingungen adaptierter Industrie-Mischstamm von Acetobacter verwendet. Als Substrat wurde mit Essigsäure denaturierter Industriesprit verwendet.

Medienzusammensetzung

| | Limit. Med. | Optimalmedium |
|---|---|---|
| | M 50 | M 60 |
| | g l$^{-1}$ | g l$^{-1}$ |
| Hefeextrakt | 2.5 | 3 |
| Mineralsalzmischung | 0.5 | 0.6 |
| (NH$_4$)$_2$HPO$_4$ (70 %), | | |
| KH$_2$PO$_4$ (23 %), | | |
| MgSO$_4$ · 7$_2$0 7 % | | |
| und Spurenelemente | | |
| | | |
| Lactat | 0.5 | 0.6 |
| Glucose | 0.2 | 0.2 |
| Citrat | 0.08 | 0.1 |
| Äthanol versuchsspez. | 15 - 100 l$^{-1}$ | |

Die Medienzubereitung erfolgte der Industriepraxis entsprechend unsteril. Als Kontaminationsschutz genügte der saure pH-Bereich. Für die wässrigen Lösungen wurde unchloriertes Trinkwasser aus dem Leitungsnetz verwendet.

Die Gärung wurde unter "Steady-state-Bedingungen" bei 30°C, einer Belüftungsrate von 0,6 vvm, unter einer Äthanolkonzentration von 70 - 85 g/l durchgeführt.

Gemäss Fig. 3 wird das erfindungsgemässe Verfahren beispielhaft an einer zweistufigen Industrieanlage mit Zellrückführung und Produktabtrennung beschrieben.

In einer ersten Stufe wird unter den Bedingungen eines Chemostaten versäuert. Das Arbeitsvolumen beträgt 10 m$^3$, wobei die Niveausteuerung das Niveau N4 anzeigt. Die Sauerstofftransferrate ist auf 55 - 60 mMol O$_2$ l$^{-1}$ entsprechend einem k$_L$a von ca. 250 h$^{-1}$ eingestellt. Die Konzentrationen betragen bei konstanter Gesamtkonzentration an Essigsäure ca. 80 g l$^{-1}$ an Äthanol 16 g l$^{-1}$. Die Zusammensetzungen werden in bekannter Weise über eine kontinuierliche Alkoholanalyse und die Substratpumpe 114 gesteuert. Eine optimale Biomassenkonzentration von 0.3 g l$^{-1}$ wird durch eine Rückführung aktiver Bakterien mittels Tangentialfiltration und bewachsenem Mediumtransfer in die Erntestufe gefördert. Die Normproduktivität r$_p$ beträgt 3.3 g l$^{-1}$ h$^{-1}$ (790 kg reine Essigsäure / 24 h).

In einer zweiten Stufe wird im Zulaufbetrieb und unter Teilerneuerung gefahren. Das Arbeitsvolumen beträgt 3 m$^3$ bei einem Niveau N3 im Erntefermenter 117. Der mikrobiologische Prozess durchläuft den nicht-optimalen Konzentrationsbereich bis zur vollständigen Umsetzung des Substrat-Äthanols zu Essigsäure von ca. 80 - 100 g l$^{-1}$, bei einer industrieüblichen Normproduktivität von r$_p$ = 1.1 g l$^{-1}$ h$^{-1}$ und entsprechend normaler Belüftungskapazität. Die pO$_2$-Sonde 110' dient als Messgeber zur Prozeßsteuerung. Der Niveaukontakt N1 der Niveausteuerung 103' im Erntefermenter 117 begrenzt das Medium-Minimalvolumen. Mittels der Niveaukontaktstrecke N1 - N2 kann die transferierte Menge an bewachsenem Medium als Inokulum aus der Hauptstufe gesteuert werden.

Zum Start der zweiten Stufe fördert die Pumpe 131 aus dem Bioreaktor 101 direkt belüftetes Medium mit aktiver aufkonzentrierter Biomasse über das Dreiwegventil 127 in den Strönungsbereich der Turbine 102'. Die Positionen der Niveaukonakte von N1 bis N2 begrenzen die Inokulumsmenge. Die Steuerung der Inokulumsmenge bewirkt die optimale Zellkonzentraion in der Hauptstufe und limitiert deren mittlere Verweilzeit.

Das Mediumniveau im Bioreaktor 101 steht über dem Niveaukonakt N4. Die Filterpumpe 131 fördert das aktive belüftete Medium aus dem Bioreaktor 101 über das Dreiwegventil 132 durch den Retentatbereich im Mikro/Ultrafilter 125 und, zwischenbelüftet durch Diffusoren 134, 134', 134" bzw. Injektoren, innerhalb von 15 Sekunden zurück in den axialen Saugbereich der Rührer-Turbine 102. Das Permeat aus dem Filter 125 fördert über die Leitung 126, umgeleitet über das Dreiwegventil 144, restalkoholhaltiges Medium über die Leitung 128 in den belüfteten Erntetank 117 als zweite Stufe zur Restoxidation. Dieser Permeatzulauf setzt bei Niveaukontakt N2 ein. Nach erreichtem Niveau N3 schaltet das Ventil 132 in Ausgangsrichtung der zweiten Stufe und das Dreiwegventil 144 in Förderrichtung des Lagertanks 146 ein. Die Pumpe 131 bleibt ausgeschaltet bis die pO$_2$-Sonde 110' den vorgegebenen Anzeigegrenzkontakt von 100 % der Steuerung 107 erreicht. Im anschliessenden Verfahrensschritt erfolgt die Klärung, indem das Gärungsessigfiltrat in den Lagertank 146 permeiert, bis zu einer Volumeneinengung eines Retentatniveaus von N1. Die Turbine 102' bleibt im Betrieb und bewirkt nach der vollständigen Substratumsetzung eine Zell-Lysis und unterstützt somit die Klärung.

Zur Produktabtrennung bzw. Aufkonzentrierung des produzierten Gärungsessigs bis 120 g l$^{-1}$: Der permeierende endfermentierte Essig aus der Mikro- bzw. Ultcafiltration fliesst über das Dreiwegventil 144 in den Lagertank 146. Die Hochdruckpumpe 136 mit einem Förderdruck von 50 - 60 bar rezirkuliert den aufzukonzentrierenden Essig retentatseitig über den Hyperfilter 138. Das Permeat mit einer Restsäure von bis 35 g 1$^{-1}$ strömt zurück in die Substrataufbereitung 115 der Hauptstufe und dient der Verdünnung des Industriealkohols.

Das erfindungsgemässe Verfahren hat einerseits den Vorteil, dass mit einer kapazitiv variablen Anlage Schwankungen im Kapazitätsbedarf eines Ganzjahresbetriebs einer Essigfabrik absgeglichen werden und dennoch betriebsüblich konzentrierter Gärungsessig kontinuierlich erzeugt wird. Auf diese Weise kann auf Lagerbestände und die dazu erforderlichen Tankanlagen zur Deckung von Bedarfsspitzen weitgehend verzichtet werden. Das Endprodukt fällt ohne zusätzliche Klärung als klares Filtrat an.

Dies ist in überraschender Weise mittels eines zweistufigen Fermentationsverfahrens gelungen, dessen Anlage aus einer Kombination von Fermentation und Tangentialfiltration im Bereich von Mikro- bis Hyperfiltration besteht.

**Patentansprüche**

1. Verfahren hoher Produktionsleistung zur submersen, kontinuierlichen Herstellung von Gärungsessig aus Äthanol in wenigstens zwei Stufen, wobei wenigstens ein Teil des Mediums zusammen mit den Essig bildenden Mikroorganismen ausserhalb eines Bioreaktors (1) von der vergorenen Maische mittels Membranfilter abgetrennt und teilweise über ein Mikro/Ultrafilter (25) mit einem Porendurchmesser von 0,001 μm bis 0,65 μm rezirkuliert wird, dadurch gekennzeichnet, dass die aktiven Miktoorganismen im Retentat unter Begasung unmittelbar nach dem Mikro/Ultrafilter (25) in den Bioreaktor (1) zurückgeführt und in hoher Konzentration von bis zu 0,3 g/l angereichert werden und mittels eines zugeschalteten Hyperfilters (38) der erzeugte Gärungsessig wenigstens partiell als Endprodukt abgetrennt und durch Hyperfiltration die Konzentration an Essigsäure im Bioreaktor (1) gesteuert wird und das Verfahren so durchgeführt wird, dass der aus dem Mikro/Ultrafilter (25) austretende Gärungsessig mittels eines Hyperfilters (38) bei 40 - 80 bar und 28-32°C auf etwa 120 g/l Essigsäure konzentriert wird und dass das Medium dem Bioreaktor (1) im Bereich der Begasungszone eines Rührers (2) entnommen wird.

2. Verfahren nach Anspruch 1, dadurch gekennzeichnet, dass das Medium vor und/oder nach und/oder in der Pumpe (31) mit Luft oder mit Sauerstoff angereicherter Luft mittels der Diffusoren (34, 34', 34") begast wird.

3. Verfahren nach den Ansprüchen 1 und 2, dadurch gekennzeichnet, dass das Retentat vor dem Eintritt in den Bioreaktor (1) erneut begast wird.

4. Verfahren nach den Ansprüchen 1 bis 3, dadurch gekennzeichnet, dass das Medium vom Austritt aus dem Bioreaktor (1) in weniger als 15 Sekunden als Retentat in den Bioreaktor (1) zurückgeführt wird.

5. Verfahren nach den Ansprüchen 1 bis 4, dadurch gekennzeichnet, dass über das Dreiweg-Magnetventil (27) und die Leitung (26) der Mikro/Ultrafilter (25) intermittierend mit Pressluft rückgespült wird.

6. Verfahren nach Anspruch 1, dadurch gekennzeichnet, dass das Permeat aus dem Hyperfilter (38) in das Substrataufbereitungsgefäss (15) des Bioreaktors (1) zurückgeführt wird.

7. Verfahren nach Anspruch 1, dadurch gekennzeichnet, dass in einer ersten Stufe in einem Bioreaktor (101) unter Steady-state-Bedingungen und in einer zweiten Stufe in einem Erntefermenter (117) bis zur vollständigen Umsetzung des Äthanols versäuert wird.

8. Anlage zur Durchführung des Verfahrens nach den Ansprüchen 1 bis 7, dadurch gekennzeichnet, dass der Bioreaktor (1) über die Dreiwegventile (24, 32) direkt mit dem begasbaren Mikro/Ultrafilter (25) verbunden ist.

9. Anlage nach Anspruch 8, dadurch gekennzeichnet, dass ein erster Begasungsdiffusor (34) vor der Pumpe (31), ein zweiter Begasungsdiffusor (34') nach der Pumpe (31) und ein weiterer Begasungsdiffusor (34") nach dem Mikro/Ultrafilter (25) vorgesehen ist.

**Claims**

1. A high productive capacity method for the submerged continuous manufacture of fermented vinegar from ethanol in at least two stages, at least one part of the medium together with the microorganisms producing the vinegar being separated outside a bioreactor (1) from the fermented mash by means of a membrane filter and being partially recirculated via a micro/ultrafilter (25) having a pore diameter of 0.001 μm to 0.65 μm, characterised in that directly after the micro/ultrafilter (25) the active microorganisms in the residue are recirculated under the application of gas into the bioreactor (1) and are concentrated in high concentrations of up to 0.3 g/l and the fermented vinegar produced is at least partially separated as an end product using a connected hyperfilter (38) and the concentration of acetic acid in acid in the bioreactor (1) is controlled by hyperfiltration and the method is carried out in such a manner that the fermented vinegar emerging from the micro/ultrafilter (25) is concentrated using a hyperfilter (38) at 40 - 80 bar and 28 - 32°C to approx 120 g/l acetic acid and the medium is removed from the bioreactor (1) in the region of the gassing zone of a stirrer (2).

2. A method according to claim 1, characericed in that the medium is gassed before and/or after and/or in the pump (31) with air or with oxygen-enriched air by means of the diffusers (34, 34', 34").

3. A method according to claims 1 and 2, characterised in that the residue is re-gassed prior to entering the bioreactor (1).

4. A method according to claims 1 to 3, characericed in that after leaving the bioreactor (1) the medium is recirculated into the bioreactor (1) as a recidue in less than 15 seconds.

5. A method according to claims 1 to 4, characeised in that backflushing with compressed air is carried out intermittently via the three-way magnetic valve (27) and the conduit (26) of the micro/ultrafilter (25).

6. A method according to claim 1, characterised in that the substrate from the hyperfilter (38) is recirculated into the substrate preparation vessel (15) of the bioreactor (1).

7. A method according to claim 1, characterised in that acidification is carried out in a first stage in a bioreactor (1) under steady state conditions and in a second stage in a harvest fermenter (117) until the ethanol is completely converted.

8. Apparatus for carrying out the method according to claims 1 to 7 characterised in that the bioreactor (1) is directly connected via the three-way valves (24, 32) with the gassable micro/ultrafilter (25).

9. Apparatus according to claim 8, characterised in that a first gassing diffuser (34) is provided upstream of the pump (31), a second gassing diffuser (34') is provided downstream of the pump (31) and a further gassing diffuser (34") is provided downstream of the micro/ultrafilter (25).

**Revendications**

1. Procédé à haut rendement pour la fabrication continue en submersion de vinaigre de la fermentation acétique à partir de l'éthanol en au moins deux étapes, au moins une partie du milieu étant séparée, conjointement avec les microorganismes formant le vinaigre, du moût fermenté, à l'extérieur d'un bioréacteur (1) au moyen de filtres à diaphragme et recyclée en partie par l'intermédiaire d'un micro/ultrafiltre (25) avec un diamètre de pores de 0,001 μm à 0,65 μm, caractérisé en ce que les micro-organismes actifs dans le rétentat sont renvoyés, avec injection de gaz et immédiatement en aval du micro/ultrafiltre (25), au bioréacteur (1) et enrichis en une forte concentration allant jusqu'à 0,3 g/l, le vinaigre de la fermentation produit étant séparé, du moins partiellement, en tant que produit final au moyen d'un hyperfiltre (38) intercalé, la concentration en acide acétique étant commandée dans le bioréacteur (1) par hyperfiltration, et le procédé étant conduit de telle façon que le vinaigre de la fermentation sortant du micro/ultrafiltre (25) est concentré, au moyen d'un hyperfiltre (38) à 40 à 80 bars et 28 à 32°C, à environ 120 g/l d'acide acétique et que le milieu est prélevé du bioréacteur (1) dans la région de la zone d'injection de gaz d'un agitateur (2).

2. Procédé selon la revendication 1, caractérisé en ce que, en amont et/ou en aval et/ou dans la pompe (31), le milieu est chargé d'air ou d'air enrichi d'oxygène au moyen des diffuseurs (34, 34', 34'').

3. Procédé selon les revendications 1 et 2, caractérisé en ce que le rétentat est de nouveau chargé en gaz avant l'entrée dans le bioréacteur (1).

4. Procédé selon l'une des revendications 1 à 3, caractérisé en ce que le milieu sortant du bioréacteur (1) est ramené en moins de 15 secondes, en tant que rétentat, dans le bioréacteur (1).

5. Procédé selon l'une des revendication 1 à 4, caractérisé en ce que, par l'intermédiaire de l'électrovanne à trois voies (27) et de la conduite (26), le micro/ultrafiltre (25) est rétrolavé de manière intermittente avec de l'air comprimé.

6. Procédé selon la revendication 1, caractérisé en ce que le perméat est ramené de l'hyperfiltre (38) au récipient de traitement du substrat (15) du bioréacteur (1).

7. Procédé selon la revendication 1, caractérisé en ce que l'acidification jusqu'à la transformation complète de l'éthanol se fait, dans une première phase, dans un bioréacteur (101) dans des conditions d'état stationnaire et, dans une seconde phase, dans un fermentateur de récolte (117).

8. Installation pour la mise en oeuvre du procédé selon les revendication 1 à 7, caractérisée en ce que, par l'intermédiaire des vannes à trois voies (24, 32), le bioréacteur (1) est relié directement au micro/ultrafiltre (25) pouvant être soumis à un courant de gaz.

9. Installation selon la revendication 8, caractérisée en ce qu'il est prévu un premier diffuseur de gaz (34) en amont de la pompe (31), un second diffuseur de gaz (34') en aval de la pompe (31) et un diffuseur de gaz supplémentaire (34'') en aval du micro/ultrafiltre (25).

FIG.1

FIG.4

EP 0 189 378 B1

FIG.2

FIG.3